Europäisches Patentamt

European Patent Office

Office européen des brevets

(11) Publication number: **0 133 358 B1**

(12) **EUROPEAN PATENT SPECIFICATION**

(45) Date of publication of patent specification: **10.04.91**    (51) Int. Cl.⁵: **A61K 35/12**

(21) Application number: **84305098.0**

(22) Date of filing: **26.07.84**

(54) Processes for the production of agents for inhibiting the proliferation of malignant tumour cells, and agents produced thereby.

(30) Priority: **28.07.83 JP 136793/83**

(43) Date of publication of application:
**20.02.85 Bulletin 85/08**

(45) Publication of the grant of the patent:
**10.04.91 Bulletin 91/15**

(84) Designated Contracting States:
**CH DE FR GB LI NL**

(56) References cited:
**EP-A- 0 087 087          EP-A- 0 102 801**
**EP-A- 0 118 286          EP-A- 0 125 125**
**EP-A- 0 133 029          EP-A- 0 136 053**

**BIOLOGICAL ABSTRACTS, vol. 66, no. 7, 1978, no. 41061, Philadelphia, PA, US; D.L. DEWEY: "The identification of a cell culture inhibitor in a tumour extract", & CANCER LETT. 1978, 4(2), 77-84**

(73) Proprietor: **KOKEN LTD.**
**No. 7, Yonban-cho**
**Chiyoda-ku Tokyo(JP)**

Proprietor: **Tajima, Tomoyuki**
**5-15, Yawata 6-chome**
**Ichikawa-shi Chiba-ken(JP)**

Proprietor: **Nagasu, Youichiro**
**No. 9-4, Chuo 3-chome**
**Yamato-shi Kanagawa-ken(JP)**

(72) Inventor: **Tajima, Tomoyuki**
**No. 5-15, Yawata 6-chome**
**Ichikawa-shi Chiba-ken(JP)**

(74) Representative: **Deans, Michael John Percy et al**
**Lloyd Wise, Tregear & CO. Norman House**
**105-109 Strand**
**London WC2R OAE(GB)**

EP 0 133 358 B1

BIOLOGICAL ABSTRACTS, vol. 65, 1st January 1978, no. 3219, Philadelphia, PA, US; D.M. BURNS et al.: "Natural occurrance of an inhibitor of mammalian cell growth in human and mouse cells of normal and tumor origin", & CANCER BIOCHEM. BIOPHYS. 1976, 1(6), 269-280

BIOLOGICAL ABSTRACTS, vol. 65, 15th March 1978, no. 34666, Philadelphia, PA, US; G.L. WRIGHT, Jr. et al.: "Isolation of a soluble tumor-associated antigen from human renal cell carcinoma by gradient acrylamide gel electrophoresis", & CANCER RES. 1977, 37(11), 4228-4232

BIOLOGICAL ABSTRACTS, vol. 65, no. 3, no. 16073, Philadelphia, PA, US; J.L. McCOY et al.: "Leukocyte migration inhibition in patients with Ewing's sarcoma by 3-M potassium chloride extracts of fresh and tissue-cultured Ewing's sarcomas", & J. NATL. CANCER INST. 1977, 59(4), 1119-1126

## Description

The present invention relates to the production of an inhibiting agent against the proliferation of malignant tumour cells.

Burns et al. have previously noted (Biological Abstracts 65 (1978), 3219) the natural occurrence of an inhibitor of mammalian growth in human and mouse cells of normal and tumour origin. Dewey (Biological Abstracts 66 (1978), 41061) describes the purification of a cell culture inhibitor, identified as spermidine, by ultra-filtration, column chromatography and preparative electrophoresis.

We have previously proposed in our European Patent Application 83304845.7 (published on 14.03.84 under No. 102801) the production of an inhibiting agent against the proliferation of malignant tumour cells comprising the steps of incubating malignant tumour cells and, after removing the malignant tumour cells, extracting an inhibiting agent from the culture medium. We have found that inhibiting agents so produced appear not to show lethal effects against normal cells, but instead to exhibit an inhibiting activity against the proliferation of malignant tumour cells and to have a lethal effect specifically to malignant tumour cells.

The present invention arises from our work seeking to obtain an agent with a significantly enhanced inhibiting effect even when provided in substantially small doses.

In accordance with a first aspect of the present invention, we provide a process for the production of an agent for inhibiting the proliferation of malignant tumour cells, characterised in that it comprises the steps of incubating malignant tumour cells in a culture medium adapted for rapid growth; transferring said cells to a serum-free culture medium adapted for extraction; removing from the culture medium solids having a molecular weight generally of $10^3$ or more; freeze-drying the remaining culture medium extracting with n-butanol as solvent; and isolating the solid matter by evaporation to dryness.

In the preferred procedure described in more detail below, the malignant tumour cells are first allowed to proliferate in a growth medium until the incubator is saturated. The cells are transferred to an extracting medium and further incubated, and are then subjected to ultra-filtration, thereby removing the malignant tumour cells. The filter culture medium is extracted with n-butanol as solvent after the freeze-drying step, and the solid matter is isolated by evaporation to dryness with heating.

The invention also extends to an agent for inhibiting the proliferation of malignant tumour cells which agent can be used after dissolving in an appropriate solvent.

Accordingly, in a second and alternative aspect of the present invention, we provide an agent for inhibiting the proliferation of malignant tumour cells, characterised in that it comprises solid matter resulting from evaporating to dryness an extraction with n-butanol as solvent from a freeze-dried, serum-free culture medium into which malignant tumour cells previously incubated in a culture medium adapted for rapid growth had been placed, and solids having molecular weights generally around or greater than $10^3$ subsequently having been removed; or a solution of such solid matter in dimethyl sulphoxide.

The tumour cells employed for the process of the present invention are preferably derived from an established cell line (hereinafter referred to "HRC") originating from a human renal carcinoma. As we shall explain below, our experiments have shown that examples of inhibiting agents produced in accordance with the present invention exhibit an inhibiting effect against the proliferation of HRC cells and with an enhanced dosage response as compared with our prior proposal referred to hereinabove.

Our experiments show that the inhibiting agent brings about a characteristic morphological change in HRC cells and scarcely any effect on human dermatic fibroblasts. Our experiments suggest that the inhibiting effect is specific to the malignant tumour cells concerned.

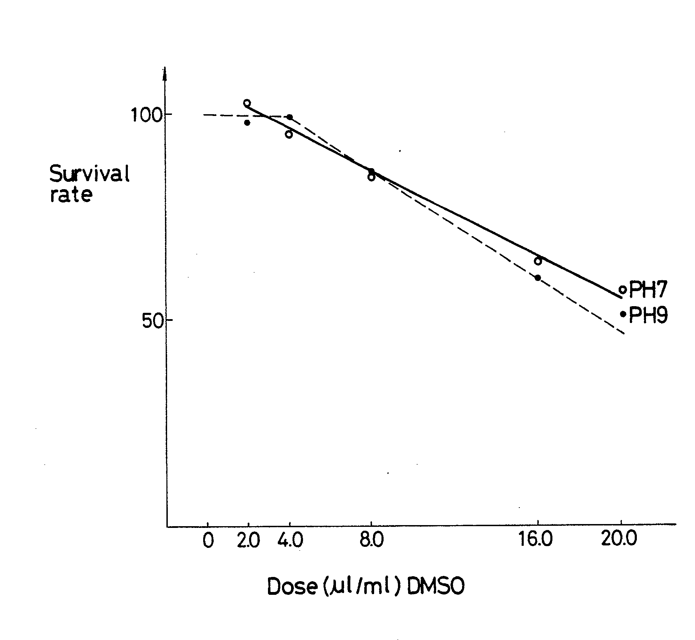

The invention is hereinafter more particularly described by way of example only with reference to an illustrative detailed example and to the accompanying drawing which illustrates in graphical form our experimental results showing the inhibiting effect when employing examples of agents in accordance with the present invention.

## Example

For this experiment we employed an establised cell line (hereinafter referred to as "HRC") originating from a human renal carcinoma.

The HRC cells were first allowed to proliferate in a culture medium adaped for rapid growth (hereinafter referred to as a "growth medium") in an incubator until the growth medium was saturated. The cells were then transferred to a second culture medium without serum (hereinafter referred to as an "extraction medium") and incubated further.

The extraction medium was subjected to ultra-filtration employing a filter adapted to fractionate at a

molecular weight of $10^3$, and thereby removing the malignant tumour cells. The extraction medium was then subjected to freeze-drying and re-dissolved in a 1 in 10 times concentration followed by extraction with n-butanol as solvent. For comparative purposes, as will be explained hereinbelow, similar extractions were performed with other organic solvents, namely hexane and methylacetate. These extractions were conducted in equal quantities, and in respect of each of the three samples, the pH value of the culture medium was adjusted to 3, 7 and 9 respectively. The extraction with the three organic solvents (and for comparative purposes culture medium after freeze-drying but before extraction, an extraction culture medium prior to both freeze-drying and extraction) were heated at 45°C to dryness in an evaporator.

We shall now describe the experiment which we have performed to confirm the inhibiting effect exhibited by the product in accordance with the present invention resulting from the above Example.

In each case, we employed the solid matter resulting from the evaporation to dryness of 60 ml of the original culture medium. Each resulting solid product was re-dissolved in 0.6 ml of dimethyl sulphoxide (hereinafter referred to as "DMSO"). In each case 15 $\mu$ l of each such re-dissolved agent was added to 15 ml of culture medium in each determination for an inhibiting effect. In a further control, DMSO alone was added. Each of 24 holes of a multi-well plate were inoculated with some $10^4$ HRC cells. Basal medium eagle (hereinafter referred to as "BME") supplemented with 10% new born calf serum was present in each hole of the plate as culture medium, the culture medium being changed every other day. The plate was incubated for 6 days, and the survival rate of HRC cells were investigated by counting at the end of this period. The experimental results are shown in Table 1 below:

|  |  | Survival rate(%) | Dose (ml) | Recovery rate(%) |
|---|---|---|---|---|
| Extraction culture medium before freeze-drying |  | 56 | 6 | 100 |
| After freeze-drying |  | 62 | 6 | 86 |
| n-Butanol | pH3 | 104 | 0.06 | – |
|  | pH7 | 68 | 0.06 | 73 |
|  | pH9 | 64 | 0.06 | 82 |
| Hexane | pH3 | 98 | 0.06 | – |
|  | pH7 | 103 | 0.06 | – |
|  | pH9 | 103 | 0.06 | – |
| Ethylacetate | pH3 | 100 | 0.06 | – |
|  | pH7 | 105 | 0.06 | – |
|  | pH9 | 97 | 0.06 | – |

As will be seen from the above, the product resulting from extraction with n-butanol at pH7 and pH9 produces an inhibition effect at a very small dose of 1/100th of that required using extraction culture medium before the freeze-drying step.

The death rate is measured by subtracting the survival rate from 100: and a recovery rate is calculated as the ratio per 100 resulting from dividing the calculated death rate of each experimental sample by the death rate for the sample using extraction culture medium before freeze-drying. It is apparent from this experiment that the active agent possessing the proliferation-inhibiting effect dissolves in n-butanol.

It would also appear that the pH has an effect on the result and that for best results the culture medium should be generally neutral or alkaline.

We have also carried out a series of dosage tests for the n-butanol extract and these are described below.

The samples employed for these tests were obtained as described previously, the pH of the extraction culture medium in 500 ml being fixed at pH7 and at pH9 respectively in two samples, followed by extraction with n-butanol and evaporation to dryness. The evaporated residue was dissolved in 2 ml of DMSO again, this solution being used for the tests described below. By calculating backwards, the quantity of the DMSO solution corresponding to 1 ml of culture medium was 4 $\mu$ l. The tests were all conducted with some $10^4$ HRC cells inoculated per 15 mm hole of a multi-well culture dish. As a culture medium in each hole we

4

used 1.5 ml of BME supplemented with 10% new born calf serum. 24 hours from such innoculation, each culture medium was exchanged for a new culture medium comprising BME supplemented with 10% new born calf serum and having a selected concentration of the DMSO solution added. After that, on every other day, the culture medium was exchanged for the same again. On the 7-8 day the cell numbers were counted to measure the proliferation rate.

In case it was the DMSO itself, rather than the n-butanol extract which was having an effect, we carried out a comparative test using DMSO without the extract. The results using DMSO alone are set out on Table 2 below:

## Table 2

| Dose (DMSO) $\mu$ 1/ml | 0 | 2.0 | 4.0 | 8.0 | 16.0 | 20.0 |
|---|---|---|---|---|---|---|
| Cell numbers X10$^4$ | 32.69 | 33.87 | 32.29 | 32.63 | 18.64 | 9.80 |

In the experiment proper using n-butanol extract in DMSO, the cell numbers were counted on the seventh day. The ratio of cell numbers to the cell numbers of the controls in which DMSO alone had been administered were calculated as a percentage survival rate. These results are set out in Table 3 below:

## Table 3

| Dose $\mu$m/ml | | 2.0 | 4.0 | 8.0 | 16.0 | 20.0 | |
|---|---|---|---|---|---|---|---|
| Conc. to orig. sol. | | 1/2 | 1 | 2 | 4 | 5 | |
| Survival rate (%) | pH7 | 103 | 95 | 85 | 64 | 57 | |
| | pH9 | 98 | 99 | 86 | 60 | 51 | |

These results are set out graphically in the accompanying single figure of the drawings. It will be seen from this graph and from Tables 2 and 3 that the solid matter resulting from extraction of the culture medium at pH7 and at pH9 with n-butanol as solvent and then evaporating to dryness produces a significant inhibiting effect against the proliferation of malignant tumour cells, and that this inhibiting effect appears directly related to and increases with the dose.

## Claims

1. A process for the production of an agent for inhibiting the proliferation of malignant tumour cells, characterised in that it comprises the steps of incubating malignant tumour cells in a culture medium adapted for rapid growth; transferring said cells to a serum-free culture medium adapted for extraction; removing from the culture medium solids having a molecular weight generally of $10^3$ or more; freeze-drying the remaining culture medium; extracting with n-butanol; and isolating the solid matter by evaporation to dryness.

2. A process according to Claim 1, further characterised in that the malignant tumour cells are derived from established cells originating from a human renal carcinoma.

3. An agent for inhibiting the proliferation of malignant tumour cells, characterised in that it comprises solid matter resulting from evaporating to dryness an extraction with n-butanol as solvent from a freeze-dried, serum-free culture medium into which malignant tumour cells previously incubated in a culture medium adapted for rapid growth had been placed, and solids having molecular weights generally around or greater than $10^3$ subsequently having been removed; or a solution of such solid matter in dimethyl sulphoxide.

**Revendications**

1. Procédé d'obtention d'un agent inhibiteur de la prolifération des cellules d'une tumeur maligne, caractérisé en ce qu'il comprend les étapes d'incubation des cellules de tumeur maligne dans un milieu de culture adapté pour une croissance rapide; transfert desdites cellules sur un milieu de culture exempt de sérum, adapté pour l'extraction; enlèvement du mileu de culture des solides ayant un poids moléculaire généralement de $10^3$ ou davantage; dessication par congélation du milieu de culture restant; extraction au n-butanol; et isolement de la matière solide par évaporation jusqu'à dessication.

2. Procédé selon la revendication 1, caractérisé en outre en ce que les cellules de la tumeur maligne sont dérivées de cellules fixées provenant d'un carcinome rénal humain.

3. Agent inhibiteur de la prolifération des cellules d'une tumeur maligne, caractérisé en ce qu'il comprend des matières solides résultant de l'évaporation jusqu'à dessication d'un extrait au n-butanol, en tant que solvant, d'un milieu de culture exempt de sérum, desséqué par congélation, dans lequel les cellules de tumeur maligne, préalablement incubées dans un milieu de culture adapté pour une croissance rapide, avaient été placées, et des solides ayant des poids moléculaires généralement voisins ou supérieurs à $10^3$ ayant été ultérieurement enlevés, ou une solution de ladite matière solide dans le diméthylsulfoxyde.

**Ansprüche**

1. Verfahren zur Herstellung eines Agens zur Hemmung der Vermehrung bösartiger Tumorzellen,
   **dadurch gekennzeichnet,**
   daß das Verfahren die folgenden Schritte umfaßt: Inkubation bösartiger Tumorzellen in einen für schnelles Zellwachstum geeigneten Kulturen-Nährboden; Übertragung der Zellen auf einen serumfreien, zur Extraktion geeigneten Kulturen-Nährboden; Entnahme von Feststoffen mit einem Molekulargewicht von im allgemeinen $10^3$ oder mehr aus dem Kulturen-Nährboden; Gefriertrocknen des verbleibenden Kulturen-Nährbodens; Extraktion mit N-Butanol; und Isolierung des Feststoffes durch Verdampfung bis zur Trockenheit.

2. Verfahren nach Anspruch 1, weiterhin
   **dadurch gekennzeichnet,**
   daß die bösartigen Tumorzellen von Zellen genommen wurden, die einem menschlichen Nierenkarzinom entstammen.

3. Agens zur Hemmung der Vermehrung bösartiger Tumorzellen,
   **dadurch gekennzeichnet,**
   daß das Agens aus Feststoff, der durch Verdampfung bis zur Trockenheit einer Extraktion mit N-Butanol als Lösungsmittel aus einem gefriergetrockneten, serumfreien Kulturen-Nährboden, in den zuvor in einen zum schnellen Wachstum geeigneten Kulturen-Nährboden inkubierte bösartige Tumorzellen gesetzt wurden, besteht sowie aus Feststoffen mit Molekulargewichten von allgemein um $10^3$ oder mehr im wesentlichen entfernt wurden; oder aus einer Lösung dieses Feststoffes in Dimethylsulfoxid.

Survival rate vs Dose (µl/ml) DMSO